(19) **Europäisches Patentamt**

**European Patent Office**

**Office européen des brevets**

(11) Publication number: **0 407 038 B1**

(12) ## EUROPEAN PATENT SPECIFICATION

(45) Date of publication of patent specification: **01.02.95**

(51) Int. Cl.6: **C07C 43/04**, C07C 41/09, C07C 41/42

(21) Application number: **90306324.6**

(22) Date of filing: **11.06.90**

(54) **Method for the preparation of dialkyl ethers.**

(30) Priority: **03.07.89 US 374830**

(43) Date of publication of application:
**09.01.91 Bulletin 91/02**

(45) Publication of the grant of the patent:
**01.02.95 Bulletin 95/05**

(84) Designated Contracting States:
**BE DE ES FR GB IT NL SE**

(56) References cited:
**US-A- 4 232 177**
**US-A- 4 918 244**

**CHEMICAL PROCESSING, February 1987, pages 27,28,30,32; W.P. STADIG: "Catalytic-distillation. Combining chemical reaction with product separation"**

(73) Proprietor: **CHEMICAL RESEARCH & LICENSING COMPANY**
**10100 Bay Area Boulevard**
**Pasadena,**
**Texas 77507 (US)**

(72) Inventor: **Smith, Lawrence A., Jr.**
**5009 Pine**
**Bellaire,**
**Texas 77401 (US)**

(74) Representative: **Cropp, John Anthony David et al**
**MATHYS & SOUIRE**
**100 Grays Inn Road**
**London, WC1X 8AL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

1. Field of the Invention

The present invention relates to the preparation of dialkyl ethers by catalytic reaction of the corresponding alcohol with itself and the concurrent distillation and separation of the product, by-products and reactants. More particularly the invention relates to the preparation of dimethyl ether, diethyl ether, dipropyl ether and dibutyl ether from the dehydration of the corresponding $C_1$ to $C_4$ alcohols.

2. Related ART

The preparation of ethers by the dehydration of alcohols using an acid is known, e.g.,

$$R\text{-OH} + H_2SO_4 \longrightarrow R\text{-O-SO}_2\text{-OH} + H_2O$$
$$R\text{-O-SO}_2\text{-OH} + H0\text{ - }R \longrightarrow R\text{-O-R} + H_2SO_4$$

Hansen in U.S., patent 3,267,156 discloses that acidic cation exchange resins effectively catalyze the selective dehydration of alcohols to ethers as exemplified by the production of diisopropyl ether.

The use of a system for concurrent reaction and distillation in a catalyst bed which also serves as the distillation structure is generically described and suggested for experimentation in other reactions in Chemical Processing, February 1987, pages 27 to 32 and in U.S. patent 4,232,177 where it is exemplified by the production of methyl tertiary butyl ether from the reaction of isobutene in a fixed $C_4$ streak with ethanol and the subsequent dissociation of the ether product to obtain essentially pure isobutene.

Other uses of the "catalytic distillation" process include the isomerization of $C_4$ alkenes as disclosed in U.S. patent 4,482,775 and a process for transetherifcation is disclosed in commonly assigned U.S. patent 4,510,336.

Systems and structures for carrying out the simultaneous reaction and distillation are disclosed variously in U.S. patents 4,215,011; 4,242,530; 4,250,052; 4,302,356; 4,307,254; 4,336,407; 4,439,350; 4,443,559; and 4,482,775 along with the above cited 4,232,177, all commonly assigned herewith. Briefly, a structure described therein is a cloth belt with a plurality of pockets spaced along the belt, which is then wound in a helix about a spacing material such as stainless steel knitted mesh. These units are then deposited in a distillation column. In addition, commonly assigned U.S. patent 4,443,559 discloses a variety of catalyst structures for this use and is herein incorporated by reference.

SUMMARY OF THE INVENTION

The present invention is a process for producing dialkyl ethers from the catalytic dehydration of the corresponding alcohol.

According to the present invention there is provided a process for the production of dialkyl ether by the dehydration of alcohol comprising the steps of:

(a) feeding a stream containing an alkyl alcohol into a feed zone of a distillation column reactor,

(b) concurrently:

(1) contacting said stream with a fixed bed solid acidic catalytic distillation structure in a distillation reaction zone thereby catalytically reacting at least a portion of said alcohol to form the corresponding dialkyl ether and water, and

(2) fractionating the resultant dialkyl ether product from water and unreacted material,

(c) withdrawing the dialkyl ether product from the distillation column reactor as a first stream containing substantially pure dialkyl ether,

(d) withdrawing water and unreacted material from the distillation column as a second stream, wherein one of said first and second streams is withdrawn as overhead, and

(e) recycling a portion of said first stream, said second stream or said first stream and said second stream to the distillation column reactor.

The alcohol preferably comprises 40 to 100% of the feed stream. If the alcohol is selected from methanol, ethanol and propanol, the first stream may be withdrawn from the distillation column reactor at a point above the distillation reaction zone and the water may be separated from the unreacted material in a distillation zone below the distillation reaction zone and withdrawn as bottoms.

If the alcohol is n-butanol and the dialkyl ether is di-n-butyl ether, the first stream containing said di-n-butyl ether may be withdrawn from the distillation column reactor at a point below the distillation reaction zone and unreacted n-butanol and water may be withdrawn in step (d) as an overhead comprising a azeotrope of n-butanol di-n-butyl ether and water.

In one preferred embodiment dimethyl-, diethyl- and dipropylethers are produced in a process comprising the steps of:

(a) feeding a stream containing an alcohol selected from the group consisting of methanol, ethanol and propanol to a distillation column reactor in a feed zone,

(b) concurrently:

(1) contacting said stream with a fixed bed solid acidic catalyst, such as an acid cation exchange resin or an acidic molecular sieve (mole sieve), catalytic distillation structure in a distillation reaction zone at a pressure of between 137.8 and 6890 kPag (20 and 1000 psig) and a temperature of between 70°C ad 300°C thereby catalytically reacting at least a portion of said alcohol to form the corresponding dialkyl ether and water, and

(2) fractionating the resultant dialkyl ether product from water and unreacted material, and

(c) withdrawing the dialkyl ether from the distillation column reactor at a point above said distillation reaction zone and returning a portion thereof to said distillation column reactor as reflux,

(d) separating said water from said unreacted materials in a distillation zone below said distillation reaction zone, and

(e) withdrawing said water from the distillation column reactor as bottoms.

In another preferred embodiment di-normal butyl ether is produced by a process comprising the steps of:

(a) feeding a stream containing n-butanol to a distillation column reactor in a feed zone,

(b) concurrently:

(1) contacting said stream containing n-butanol with a fixed bed solid acidic catalytic distillation structure in a distillation reaction zone at a pressure of between 0 and 689 kPag (0 and 100 psig) and a temperature of between 90°C and 150°C thereby catalytically reacting at least a portion of the n-butanol to from di-n-butyl ether, and

(2) fractionating the resultant di-n-butyl ether product from water and unreacted material,

(c) withdrawing di-n-butyl ether from said distillation column reactor at a point below said distillation reaction zone

(d) withdrawing an overhead stream comprising an azeotrope of n-butanol, di-n-butyl ether and water,

(e) condensing and accumulating said overhead stream as a two phase mixture having a top phase mostly n-butanol and di-n-butyl ether and a bottom phase comprising water,

(f) withdrawing said bottom phase comprising mostly water and recovering any alcohol contained therein, and

(g) withdrawing said top phase containing mostly n-butanol and di-n-butyl ether and returning a portion thereof to said distillation column reactor as reflux.

In all embodiments the temperature in the distillation reaction zone is preferably the boiling point of the reaction mixture at the pressure conditions therein.

The alcohol stream in the distillation reaction zone as well as the products are in both vapor and liquid phase, just as the materials in a standard packed distillation column. As a result the reaction may proceed in both phases with the apparent reaction rate being higher than expected. Because the reaction is occurring concurrently with the distillation, the initial reaction product is removed from the reaction zone as quickly as it is formed and thus cannot contribute to the reverse reaction (Le Chatelier's Principle).

The temperature in the column is determined by the boiling point of the liquid mixture present at any given pressure. The temperature in the lower portions of the column will reflect the constitution of the material in that portion of the column, which will be higher than the overhead; that is, at constant pressure a change in the temperature indicates a change in the composition in the column. To change the temperature the pressure is changed. Temperature control in the reaction zone is thus controlled by the pressure with the addition of heat (the reactions being endothermic) only causing more boil up. By increasing the pressure the temperature is increased, and vice versa.

BRIEF DESCRIPTION OF THE DRAWING

Fig. 1    shows a schematic representation of the process of the present invention for producing dimethyl-, diethyl- and dipropyl ethers.

Fig. 2    shows a schematic representation of the process of the present invention for producing di-n-butyl ether.

DETAILED DESCRIPTION OF THE PREFERRED EMBODIMENTS

1. Catalyst and Distillation Structure

Molecular sieves are porous crystalline, three-dimensional alumina-silicates of the zeolite mineral group. The crystal skeleton is composed of silicon and aluminum atoms each surrounded by four oxygen atoms to form a small pyramid or tetrahedron (tetrahedral coordination). The term molecular sieve can be applied to both naturally occurring zeolites and synthetic zeolites. Naturally occurring zeolites have irregular pore size and are not generally considered as equivalent to synthetic zeolites. In the present invention, however, naturally occurring zeolites are acceptable so long as they are substantially pure. The balance of the present discussion shall be directed to the synthetic zeolites with the understanding that natural zeolites are considered equivalent thereto as indicated above, i.e. in so far as the natural zeolites are the functional equivalents to the synthetic zeolites.

Usually synthetic zeolites are prepared in the sodium form, that is, with a sodium cation in close proximity to each aluminum tetrahedron and balancing its charge. To date four principal types of molecular sieves have been reported, A, X, Y and L erionite, omega and mordenite. The A type have relative small pore size. By the term pore size is meant the effective pore size (diameter) rather than the free pore size (diameter). Types X and Y have larger pore size (approximately 10 Å.) and differ as to the range of ratio of $Al_2O_3$ to $SiO_2$ as:

Type X--------------- $Al_2O_3/2.0$-$3.0$ $SiO_2$
Type Y--------------- $Al_2O_3$ /$3.0$-$6.0$ $SiO_2$

Type L and other types listed have still higher ratios of $SiO_2$ to $Al_2O_3$

The mole sieve catalysts employed in the present invention are the acid form mole sieves or exhibit acidic characteristics. The acid form of the mole sieves is commercially available, but also may be prepared by treating the mole sieves with acid to exchange Na for hydrogen. Another method to produce the acid form is to treat the mole sieve with decomposable cations (generally ammonium ions) to replace Na with the decomposable ions and thereafter to heat the mole sieve to decompose the cation leaving the acid form. Generally the Na form mole sieve is treated with ammonium hydroxide to remove the Na and thereafter the mole sieve is heated to a temperature of about 350°C to remove the ammonia. The removal of $Na^+$ ions with $NH^+_4$ is more easily carried out than with multivalent ions as described below and these catalysts are generally more active, but less stable to heat than the multivalent cation exchange forms. Mole sieves, which have had their alkali metal reduced to low levels by partial treatment with $NH^+_4$ and partial multivalent metal cation exchange, possess increased activity and increased stability.

In addition to mole sieves which are acidic according to the Bronsted Theory, those mole sieves which exhibit acidic characteristics under the Lewis Theory, for example, calcium exchanged mole sieves are suitable for the present reaction. By exchanging the univalent cations (e.g. $Na^+$ ) with multivalent cation, strong ionic activity is imparted. The ratio of $SiO_2$ : $Al_2O_3$, valence and radius of the cation and the extent of exchange all affect the catalyst activity. In general activity increases with (1) increased $SiO_2$ : $Al_2O_3$ ratio, (2) decreased cation radius and an increase in cation valence. The effect of replacing univalent ions (e.g. $Na^+$) with bivalent (e.g. $Ca^{++}$) is much greater than replacing the bivalent ions with cations of greater valence.

The various types of mole sieves having reduced alkali metal content are characterized as the acid form molecular sieve and are all contemplated as useful in the present invention.

It would appear that the pore size within the crystal lattice may affect selectivity. According to one theory of molecular sieve catalytic activity, zeolite catalysis occurs primarily inside the uniform crystal cavities, consequently zeolitic catalyst activity depends on the number of aluminum atoms in the crystal and thus on the chemical composition of the crystal. Moreover, these catalytic sites are fixed within the rigid structure of the crystal, so that access to site can be altered by altering the structure of the crystal.

The acid form mole sieves are generally produced and available as particles in the range of < 10 $\mu$m (powders) to 5.08 mm (0.2 inch) in diameter (beads).

In this form the mole sieves form too compact a bed and will not function adequately in a distillation, since there is a very large pressure drop through the bed and the free flow of internal reflux and rising vapor is impeded. Mole sieves in the shape of conventional distillation structures, such as rings, saddles, and the like may be used in the present invention. The particulate mole sieves may be employed by enclosing them in a porous container such as cloth, screen wire or polymeric mesh. The material used to make the container must be inert to the reactants and conditions in the reaction system. The cloth may be

4

any material which meets this requirement such as cotton, fiber glass, polyester, nylon and the like. The screen wire may be aluminum, steel, stainless steel and the like. The polymer mesh may be nylon, teflon or the like. The mesh or threads per inch of the material used to make the container is such that the catalyst is retained therein and will not pass through the openings in the material. Particles of about 0.15 mm size or powders may be used and particles up to about 6.35mm (1/4 inch) diameter may be employed in the containers.

Suitable acid cation exchange resins include those which contain sulfonic acid groups, and which may be obtained by polymerization or copolymerization of aromatic vinyl compounds followed by sulfonation. Examples of aromatic vinyl compounds suitable for preparing polymers or copolymers are: styrene, vinyl toluene, vinyl naphthalene, vinyl ethylbenzene, methyl styrene, vinyl chlorobenzene and vinyl xylene. A large variety of methods may be used for preparing these polymers; for example, polymerization alone or in admixture with other monovinyl compounds, or by crosslinking with polyvinyl compounds; for example, with divinyl benzene, divinyl toluene, divinyl phenylether and others. The polymers may be prepared in the presence or absence of solvents or dispersing agents, and various polymerization initiators may be used, e.g., inorganic or organic peroxides, persulfates, etc.

The sulfonic acid group may be introduced into these vinyl aromatic polymers by various known methods; for example, by sulfating the polymers with concentrated sulfuric and chlorosulfonic acid, or by copolymerizing aromatic compounds which contain sulfonic acid groups (see e.g., US Pat. No. 2,366,007). Further sulfonic acid groups may be introduced into the polymers which already contain sulfonic acid groups; for example, by treatment with fuming sulfuric acid, i.e., sulfuric acid which contains sulfur trioxide. The treatment with fuming sulfuric acid is preferably carried out at 0 to 150° C and the sulfuric acid should contain sufficient sulfur trioxide so that it still contains 10 to 50% free sulfur trioxide after the reaction. The resulting products preferably contain an average of 1.3 to 1.8 sulfonic acid groups per aromatic nucleus. Particularly, suitable polymers which contain sulfonic acid groups are copolymers of aromatic monovinyl compounds with aromatic polyvinyl compounds, particularly, divinyl compounds, in which the polyvinyl benzene content is preferably 1 to 20% by weight of the copolymer (see, for example, German Patent Specification 908,240). The ion exchange resin is generally used in a granular size of about 0.25 to 1 mm, although particles from 0.15 mm up to about 2 mm may be employed. The finer catalysts provide high surface area, but also result in high pressure drops through the reactor. The macroreticular form of these catalysts have much larger surface area exposed and limited swelling which all of these resins undergo in a non-aqueous hydrocarbon medium compared to the gelular catalysts.

The container employed to hold the catalyst particles may have any configuration, such as the pockets disclosed in the commonly assigned patents above or the container may be a single cylinder, sphere, doughnut, cube, tube or the like.

Each container containing a solid catalytic material comprises a catalyst component. Each catalyst component is intimately associated with a spacing component which is comprised of at least 70 volume % open space up to about 95 volume % open space. This component may be rigid or resilient or a combination thereof. The combination of catalyst component and spacing component form the catalytic distillation structure. The total volume of open space for the catalytic distillation structure should be at least 10 volume % and preferably at least 20 volume % up to about 65 volume %. Thus, desirably the spacing component or material should comprise about 30 volume % of the catalytic distillation structure, preferably about 30 volume % to 70 volume %. Resilient materials are preferred. One suitable such material is open mesh knitted stainless wire, known generally as demister wire or an expanded aluminum. Other resilient components may be similar open mesh knitted polymeric filaments of nylon, teflon and the like. Other materials such as highly open structures foamed material, e.g., reticulated polyurethane foam (rigid or resilient) may be formed in place or applied around the catalyst component.

In the case of larger catalyst components such as from about 6.35 mm (1/4 inch) to 12.7 mm (1/2 inch) pellets, spheres, pills and the like, each such larger component may be individually intimately associated with or surrounded by the spacing component as described above.

It is not essential that the spacing component, entirely cover the catalyst component. It is only necessary that the spacing component intimately associated with the catalyst component will act to space the various catalyst components away from one another as described above. Thus, the spacing component provides in effect a matrix of substantially open space in which the catalyst components are randomly but substantially evenly distributed.

A preferred catalytic distillation structure for use herein comprises placing the mole sieve or cation exchange resin particles into a plurality of pockets in a cloth belt, which is supported in the distillation column reactor by open mesh knitted stainless steel wire by twisting the two together in a helical form. This allows the requisite flows and prevents loss of catalyst. The cloth may be any material which is inert in the reaction. Cotton or linen are useful, but fiber glass cloth or "Teflon" cloth are preferred. Commonly assigned U.S. Patent Nos. 4,443,559 and 4,250,052 disclose a variety of catalyst structures for this use and are incorporated herein.

In the following examples the catalyst packing consisted of bags in the form of a fiber glass cloth belt approximately 152.4 mm (six inches) wide with narrow pockets approximately 19.05 mm (3/4 inch) wide sewn across the belt. The pockets are spaced about 6.35 mm (1/4 inch) apart. These pockets are filled with the catalyst particles to form approximately cylindrical containers, and the open ends are then sewn closed to confine the particles. This belt is then twisted into a helical form to fit inside the column. Twisted in with the belt is also a strip of an open mesh knitted stainless steel wire, which serves to separate the molecular sieve filled cloth pockets and provide a passage for vapor flow.

The wire mesh provides the support for the catalyst (belt) and provides some degree of vapor passage through the catalyst particles, which otherwise form a very compact bed which has a high pressure drop. Thus, the down flowing liquid is in intimate contact with the rising vapors in the column.

In commercial-scale operations, it is contemplated, catalyst packing would be made up of alternating layers of mole sieve filled cloth belts similar to the ones described above, and a spacing material which could be of any convenient, suitable substance, such as a corrugated wire screen or wire cloth or a knitted wire mesh. The layers would be arranged vertically or horizontally. For simplicity of fabrication and for better distribution of vapor flow passages, a vertical orientation is preferred. The height of a section of this packing could be of any convenient dimension, from a few inches to several feet. For ease of assembly and installation, the packing would be made into sections of the desired shape and size, each section fastened together with circumferential bands of tie wires depending on its size and shape. A complete assembly in a column would consist of several sections, arranged in layers, with possibly the orientation of the catalyst-filled belts turned at right angles in successive layers to improve liquid and vapor flow distribution.

2. Process Description

The manner of operating the distillation column reactor, i.e., the severity of the conditions, is determined by the operator to achieve the desired result. The pressure of the column is increased until the desired extent of reaction is obtained. For the $C_1$ to $C_3$ alcohol conversion this pressure should be in the range of 137.8 to 6890 kPag (20-1000 psig) where the temperature will be from 70° to 300°C with the preferred pressure range being between 344.5 to 4134 kPag (50 to 600 psig) at temperatures of 90° to 250°C. For the n-$C_4$ alcohol feed the pressure range is between 0 to 689 kPag (0 to 100 psig) yielding temperatures in the range of between 90° to 150°C with the preferred pressure range being between 0 to 275.6 kPag (0 to 40 psig) at temperature of between 90° and 120°C.

Since there is one mole of water produced for each mole of dialkyl ether the reaction system is somewhat self diluting. However, in the case of the butyl ether production it may be desirable to have a diluent present as an azeotroping agent for water. Such a diluent should be one having a lower boiling point than the butyl ether and be inert in the reactor, for example n-butane.

The reaction system can be described as heterogeneous since the catalyst remains as a distinct entity. The catalyst may be employed in such conventional distillation packing shapes, as Rashig rings, Pall rings, saddles or the like. Similarly, the catalyst may be employed in granular or bead form as described herein above.

Bulk type liquid phase reactions have as one problem the control of the temperature. The distillation avoids the problem entirely. The success of the catalytic distillation approach lies in an understanding of the principles associated with distillation. First, because the reaction is occurring concurrently with distillation, the initial reaction product, the dialkyl ether, is removed from the reaction zone nearly as quickly as it is formed. This removal of the ether minimizes decomposition of the ether which is catalyzed by the same catalyst. Second, because the dialkyl ether is boiling, the temperature of the reaction is controlled by the boiling point of the mixture in the reactor at the system pressure. The heat of the reaction, which is endothermic, simply consumes more boil up, but no change in temperature. That is, if the heat is added in excess, there is no harm done since the excess will only result in more boil up. Third, the reaction has an increased driving force because the reaction products have been removed and cannot contribute to the reverse reaction (Le Chatelier's Principle).

As a result, a great deal of control over the rate of reaction and distribution of products can be achieved by regulating the system pressure. Also, adjusting the throughput (residence time = liquid hourly space velocity$^{-1}$) gives further control of product distribution.

A reflux is preferably included in the system. The reflux ratio could vary over the rate of 0.5 to 25:1. In practice, the higher ratio may be used to compensate for a short catalyst bed such as required for experimental work. In commercial size units the catalyst bed would be provided so that lower reflux and hence higher unit productivity could be obtained, e.g., 0.5 to 2:1.

A. Production of Dimethyl-, Diethyl- and Dipropyl Ethers

In this reaction both the ether product and water must be removed to force the reaction to completion. The water is removed as bottoms, with possible alcohol contamination, and the dialkyl as overhead. The recovery of the product in this embodiment is relatively simple since water does not form an azeotrope with either dimethyl-, diethyl- or dipropyl ether.

Another advantage of this embodiment is that the alcohol can be substantially removed from the water leaving as bottoms by operating the lower portion of the tower to distill the alcohol from the water. This may reduce the passthrough in the distillation tower-reactor, which is more than offset by the elimination of other distillation towers to recover the alcohol from the water once it leaves the reactor.

Either the acid cation exchange resins or molecular sieves described above may be used for the reaction. However, the temperature stability of these resins is not as satisfactory as the molecular sieves which are preferred for this service.

Example 1

Production of Dimethyl Ether

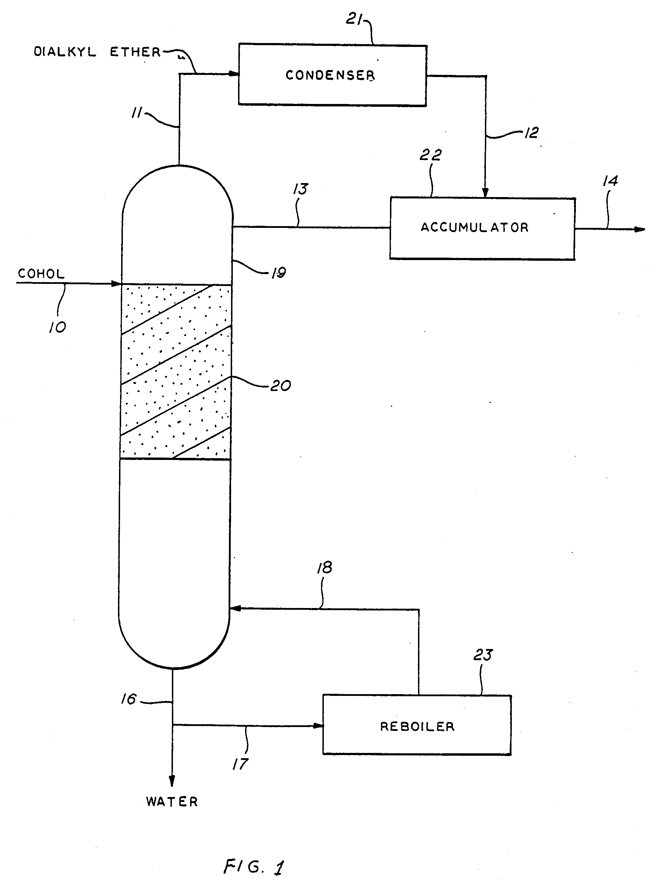

Operation of the particular embodiment for converting the $C_1$ to $C_3$ alcohols to the corresponding dialkyl ethers can be readily understood from a consideration of Fig. 1. The reactor is a 18.3 m (60 foot) tower having the following configuration:

bottom 7.6 m (25 feet), 101.6 mm (4 inch) diameter packed with 15.9 mm (5/8 ") Pall rings;

second 7.6 m (25 feet), 76.2 mm (3 inch) diameter containing the mole sieve and supported as described above;

top 3.0 m (10 feet), 101.6 mm (4 inch) diameter packed with 15.9 mm (5/8 ") Pall rings.

The catalyst packing consisted of bags in the form of a cloth belt approximately 152.4 mm (six inches) wide with narrow pockets approximately 19.05 mm (3/4 inch) wide sewn across the belt. The pockets are spaced about 6.35 mm (1/4 inch) apart. These pockets are filled with catalyst particles 1.59 mm (1/16 inch) mole sieve pellets) to form approximately cylindrical containers, and the open ends are sewn closed to confine the particles. This belt is then twisted into a helical form to fit inside the 76.2 mm (three inch) column. Twisted in with the belt is also a strip of open mesh knitted stainless steel wire which serves to separate the mole sieve filled pockets and provide a passage for vapor flow. The mole sieve used was Union Carbide Y-82 which is a mole sieve which has been exchanged with ammonia and heated to provide the acid form. Amberlyst XE 383, a product of Rohm and Haas of Philadelphia, Pennsylvania, is a suitable catalyst resin. This is a heat stabilized resin.

The wire mesh provides the support for the catalyst (belt) and provides some degree of vapor passage through the catalyst pellets, which otherwise form a very compact bed which has a high pressure drop. Thus, the down flowing liquid is in intimate contact with the rising vapors in the column.

Referring to Fig. 1, the embodiment is illustrated by the production of dimethyl ether (DME). Methanol 10 is fed at the upper end of (or somewhat above) the catalyst bed 20 in reactor 19. The exact location of the feed is not critical, however, location of feed at different points will, of necessity, result in changes in the conditions to obtain the requisite contact of the feed with the catalyst and the concurrent fractionation. The location of feed to optimize the function of the process is easily carried out by those operating in the field. Moreover, the characteristics of each individual feed needs to be ascertained for optimum operation. The overhead 11 comprises dimethyl ether. which is condensed in condenser 21 and fed via 12 to accumulator 22; a portion of which is returned as reflux via 13 and a portion recovered via 14.

The bottoms portion are recovered via 16 and are primarily water. A reboiler 23 is provided to recycle a portion of the bottoms via 17 and 18. To illustrate this embodiment a typical operation using 99 + % methanol feed is provided. The conditions and character of each stream are provided below in tabular form in TABLE I.

The overhead stream 14 was fractionated in a 7.6 m (25 foot) 101.6 mm (4") column containing 15.9 mm (5/8 ") Pall rings and a 99.9 + % DME product recovered. This fractionation can be carried out in a longer conventional distillation zone above the catalyst packing, such that a 99.9 + % DME stream could be recovered from a single tower.

7

EP 0 407 038 B1

## TABLE I

| PRODUCTION OF DIMETHYL ETHER | | | | |
|---|---|---|---|---|
| Stream | 10 | 11 & 12 | 13 & 14 | 16 |
| Component kg/hr (lb/hr) | | | | |
| Methanol | 5.8 (12.7) | 0.8 (1.7) | 0.8 (1.7) | -- |
| Dimethyl Ether | --- | 3.6 (7.9) | 3.6 (7.9) | -- |
| Water | --- | --- | --- | 1.4 (3.1) |

Example 2

Production of Diethyl Ether

In another run using a 1.8 m (six foot) 25.4 mm (1") diameter bench reactor containing 0.6 m (2 feet) of the described catalyst packing (about three of the pockets containing about 80 grams of the mole sieve). 0.6 m (Two feet) of conventional distillation packing was placed above and below the catalyst packing. The feed was C.P. grade ethanol, fed 152.4 mm (six inches) above the catalyst bed. The conditions and results of operation at several pressures are reported in TABLE II. Intermittent analysis of the overhead for light ends indicated the samples contained about 1.0% light ends (e.g. <0.1 ethylene.)

## TABLE II

| PRODUCTION OF DIETHYL ETHER | | | | |
|---|---|---|---|---|
| Pressure, kPag (psig) | 1791 (260) | 2239 (325) | 2377 (345) | 2515 (365) |
| Temperature, $^\circ$C ($^\circ$F) | | | | |
| Overhead | 140 (284) | 140 (284) | 173 (344) | 149 (300) |
| upper Cat. Bed | 171 (340) | 182 (360) | 184 (364) | 182 (360) |
| Lower Cat. Bed | 173 (344) | 184 (364) | 186 (366) | 187 (368) |
| Bottoms | 178 (352) | 191 (376) | 191 (376) | 200 (392) |
| Overhead Recovery | | | | |
| Rate g/hr | 30 | 30 | 18 | 30 |
| Reflux Ratio | 50/2 | 50/2 | 50/2 | 50/2 |
| Overhead Analysis, wt % | | | | |
| Ethanol | 49.2 | 53.4 | 19.6 | 32.2 |
| Diethyl Ether | 57.1 | 46.6 | 80.4 | 66.8 |
| Production Rate | | | | |
| g DEE/g cat./hr | 0.21 | 0.17 | 0.18 | 0.25 |
| Conversion % | 57.1 | 46.4 | 80.4 | 66.8 |
| Selectivity % | -------------About 98%------------ | | | |

B. Production of Di-n-butyl Ether

As in the conversion of the $C_1$ to $C_3$ alcohols both the ether product and water must be removed to force the reaction to completion. The water is removed as an azeotrope with butanol and the butyl ether in the overhead. Quite fortuitously, when the overhead is condensed and accumulated, two phases result, i.e., a lower phase which is about 95% water and an upper phase which contains only about 5% water. The upper phase is preferably recycled to the reactor as reflux and the lower water phase removed for purification and disposal.

8

Example 3

Production of Di-n-Butyl Ether

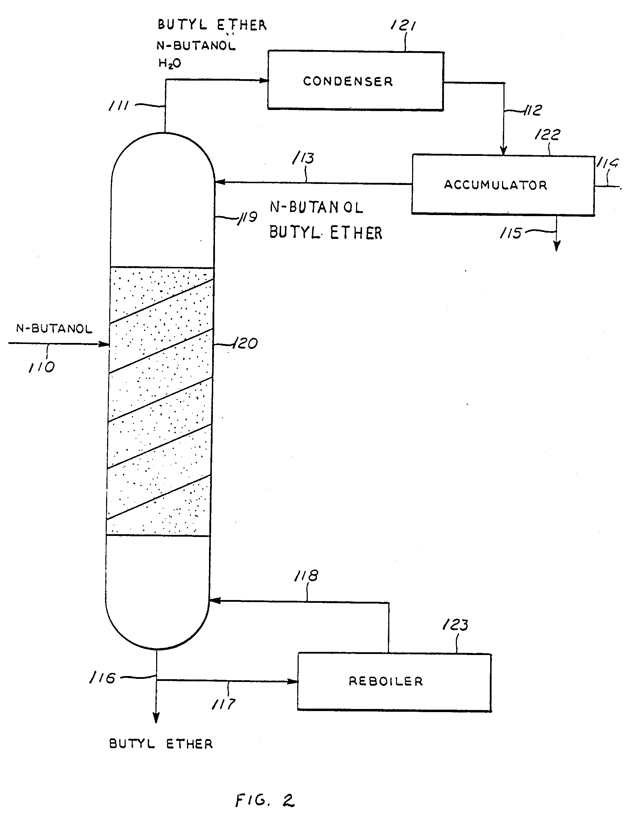

Operation of the embodiment for the production of di-n-butyl ether can be readily understood from a consideration of Fig. 2. The catalyst used was Amberlyst 15, a product of Rohm & Haas of Philadelphia, Pennsylvania, placed into the pockets on the belt and wound with the stainless steel wire mesh as described above. The catalyst was placed in approximately the middle 4.6 m (15 feet) of a 101.6 mm (four inch) diameter 10.7 m (35 foot) long column supported by conventional Pall rings and having the top portion containing the Pall rings.

Referring again to Fig.2 n-butanol 110 is fed at approximately 1/4 of the length down the catalyst bed 120 in reactor 119. The exact location of the feed is not critical, however, it is preferred that the feed be made into the catalyst packing to initiate the reaction. The overhead 111 comprises an azeotrope of n-butanol, butyl ether, and water. This mixture is condensed in condenser 121 and fed via 112 to accumulator 122 where the phases separate. The upper layer is principally n-butanol and butyl ether, a portion of which is returned as reflux via 113 and a portion recovered for recycle or other use. The water phase (bottom phase) is removed via line 115 and can be fractionated to recover the alcohol. The bed temperature is in the range of 110 to 115°C.

The bottoms portion are recovered via 116 and are, high purity di-n-butyl ether. A reboiler is provided to recycle a portion of the bottoms via 117 and 118. To illustrate this embodiment a typical operation using 100% n-butanol feed is provided. The conditions and character of each stream are provided below in TABLE III.

## TABLE III
### PRODUCTION OF DI-N-BUTYL ETHER

| Pressure | Atmospheric | | | |
|---|---|---|---|---|
| Temperature, °C | | | | |
| Overhead | 91 | | | |
| Bottoms | 142 | | | |
| | | | | |
| Stream | 111 & 112 | 113 & 114 | 115 | 116 |
| Component | | | | |
| n-butanol | 34.6 | 46.0 | 4.9 | --- |
| Butyl ether | 34.5 | 49.0 | 0.3 | 100.0 |
| Water | 29.0 | 5.0 | 94.8 | --- |

EXAMPLE 4

PRODUCTION OF BUTYL ETHER WITH TOTAL REFLUX

In another run using the 0.7 m (35 foot) tower containing 4.6 m (15 feet) of the described catalyst packing, n-butanol was fed to the tower which was operated under total reflux for several hours. the bottoms analysis showed 20 wt% butyl ether and 80 wt% n-butanol. The condensed overhead had a discrete water phase.

**Claims**

1. A process for the production of dialkyl ether by the dehydration of of alcohol comprising the steps of:
   (a) feeding a stream containing an alkyl alcohol into a feed zone of a distillation column reactor,
   (b) concurrently:
   (1) contacting said stream with a fixed
   bed solid acidic catalytic distillation structure in a distillation reaction zone thereby catalytically reacting at least a portion of said alcohol to form the corresponding dialkyl ether and water, and

(2) fractionating the resultant dialkyl ether product from water and unreacted material,

(c) withdrawing the dialkyl ether product from the distillation column reactor as a first stream containing substantially pure dialkyl ether,

(d) withdrawing water and unreacted material from the distillation column as a second stream, wherein one of said first and second streams is withdrawn as overhead, and

(e) recycling a portion of said first stream, said second stream or said first stream and said second stream to the distillation column reactor.

2. A process according to claim 1 wherein said alcohol comprises 40 to 100% of the feed stream.

3. A process according to claim 1 or claim 2 wherein said alcohol is selected from the group consisting of methanol, ethanol or propanol, and: (1) said first stream is withdrawn from said distillation column reactor at a point above said distillation reaction zone, (2) said water is separated from said unreacted material in a distillation zone below said distillation reaction zone and withdrawn as bottoms.

4. A process according to claim 3 wherein said solid acidic catalytic distillation structure consists of acidic molecular sieve.

5. A process according to claim 3 or claim 4 wherein the pressure in the column is in the range of 137.8 to 6890 kPag (20 to 1,000 psig).

6. A process according to any one of claims 3 to 5 wherein the temperature is the range of 70° to 300°C.

7. Tile process according to any one of claims 3 to 6 wherein the pressure in the column is in the range of 344.5 to 4134 kPag (50 to 600 psig), and the temperature is in the range of 90° to 250°C.

8. A process for the production of dimethyl-, diethyl- or dipropyl ether according to any one of claims 1 to 7 comprising the steps of:

(a) feeding a stream containing an alkyl alcohol selected from the group consisting of methanol, ethanol or propanol to a distillation column reactor into a feed zone,

(b) concurrently:

(1) contacting said stream containing said alcohol with a fixed bed solid acidic catalytic distillation structure in a distillation reaction zone at a pressure of between 137.8 and 6890 kPag (20 and 1,000 psig) and a temperature of between 70° and 300°C thereby catalytically reacting at least a portion of said alcohol to form the corresponding dialkyl ether and water, and

(2) fractionating the resultant dialkyl ether product from water and unreacted material,

(c) withdrawing said dialkyl ether product from said distillation column reactor at a point above said distillation reaction zone and returning a portion thereof to said distillation column reactor as reflux,

(d) separating said water from said unreacted materials in a distillation zone below said distillation reaction zone, and

(e) withdrawing said water from said distillation column reactor as bottoms.

9. A process according to claim 1 or claim 2 wherein said alcohol is n-butanol and said dialkyl ether is di-n-butyl ether, and: (1) said first stream containing said di-n-butyl ether is withdrawn from said distillation column reactor at a point below said distillation reaction zone: and (2) unreacted n-butanol and water are withdrawn in step (d) as an overhead comprising an azeotrope of n-butanol, di-n-butyl ether and water.

10. A process according to claim 9 wherein the pressure in the column is in the range of 0 to 689 kPag (0 to 100 psig).

11. A process according to claim 8 or claim 9 wherein the temperature is in the range of 90° to 150°C.

12. A process according to any one of claims 9 to 11 wherein the pressure in the column is in the range of 0 to 275.6 kPag (0 to 40 psig), and the temperature is in the range of 90° to 120°C.

13. A process according any one of claims 1 to 12 wherein the temperature in said distillation reaction zone is the boiling point of the reaction mixture at the pressure conditions therein.

**14.** A process for the production of di-n-butyl ether according to any one of claims 9 to 13 comprising the steps of:

(a) feeding a stream containing n-butanol to a distillation column reactor into a feed zone,

(b) concurrently:

(1) contacting said stream containing said n-butanol with a fixed bed solid acidic catalytic distillation structure in a distillation reaction zone at pressure of between 0 to 689 kPag (0 and 100 psig) and a temperature of between 90° and 150°C thereby catalytically reacting at least a portion of said n-butanol to form di-n-butyl ether and water, and

(2) fractionating the resultant di-n-butyl ether product from water and unreacted material,

(c) withdrawing di-n-butyl ether product from said distillation column reactor at a point below said reaction distillation zone,

(d) withdrawing an overhead stream comprising an azeotrope of n-butanol, di-n-butyl ether and water,

(e) condensing and accumulating said overhead stream as a two phase mixture having a top phase mostly n-butanol and di-n-butyl ether and a bottom phase comprising water,

(f) withdrawing said bottom phase comprising mostly water and recovering any alcohol contained therein, and

(g) withdrawing said top phase containing mostly n-butanol and di-n-butyl ether and returning a portion thereof to said distillation column reactor as reflux.

## Patentansprüche

**1.** Verfahren zur Herstellung von Dialkylether durch Dehydratation von Alkohol, bei dem man

a) einen Strom, der einen Alkylalkohol enthält, in eine Beschickungszone eines Destillationssäulenreaktors einspeist;

b) gleichzeitig

(1) diesen Strom mit einer festen, sauren, katalytischen Destillationsstruktur in Form eines Festbetts in einer Destillationsreaktionszone in Kontakt bringt und dadurch katalytisch zumindest einen Teil des Alkohols zur Umsetzung bringt, um den entsprechenden Dialkylether und Wasser zu bilden und

(2) das dabei entstehende Dialkyletherprodukt von Wasser und nicht umgesetztem Material fraktioniert;

c) das Dialkyletherprodukt aus dem Destillationssäulenreaktor als ersten Strom, der im wesentlichen reinen Dialkylether enthält, abzieht,

d) Wasser und nicht umgesetztes Material aus der Destillationssäure als zweiten Strom abzieht, wobei entweder der erste oder der zweite Strom abdestilliert wird, und

e) einen Teil des ersten Stroms, des zweiten Stroms oder des ersten und des zweiten Stroms zum Destillationssäulenreaktor zurückfuhrt.

**2.** Verfahren nach Anspruch 1, bei dem der Alkohol 40 bis 100 % des Beschickungsstroms ausmacht.

**3.** Verfahren nach Anspruch 1 oder 2, bei dem der Alkohol aus der aus Methanol, Ethanol oder Propanol bestehenden Gruppe ausgewählt wird und (1) der erste Strom an einem Punkt oberhalb der Destillationsreaktionszone aus dem Destillationssäulenreaktor abgezogen wird und (2) das Wasser in einer Destillationszone unterhalb dieser Destillationszone von dem nicht umgesetzten Material abgetrennt und als Bodenstrom abgezogen wird.

**4.** Verfahren nach Anspruch 3, bei dem die feste, saure, katalytische Destillationsstruktur aus einem sauren Molekularsieb besteht.

**5.** Verfahren nach Anspruch 3 oder 4, bei dem der Druck in der Säule im Bereich von 137,8 bis 6890 kPa g (20 bis 1.000 psig) liegt.

**6.** Verfahren nach einem der Ansprüche 3 bis 5, bei dem die Temperatur im Bereich von 70 bis 300°C liegt.

**7.** Verfahren nach einem der Ansprüche 3 bis 6, bei dem der Druck in der Säule im Bereich von 344,5 bis 4134 kPa g (50 bis 600 psig) und die Temperatur im Bereich von 90 bis 250°C liegt.

**8.** Verfahren zur Herstellung von Dimethyl-, Diethyl-, oder Dipropylether nach einem der Ansprüche 1 bis 7, bei dem man

a) einen Strom, der einen beliebigen, aus der aus Methanol, Ethanol oder Propanol bestehenden Gruppe ausgewählten Alkohol enthält, in eine Beschickungszone eines Destillationssäulenreaktors einspeist;

b) gleichzeitig

(1) diesen den Alkohol enthaltenden Strom mit einer festen sauren katalytischen Destillationsstruktur in Form eines Festbetts in einer Destillationsreaktionszone bei einem Druck zwischen 137,8 und 6890 kPa g (20 und 1.000 psig) und einer Temperatur zwischen 70 und 300°C in Kontakt bringt und dadurch katalytisch zumindest einen Teil des Alkohols zur Umsetzung bringt, um den entsprechenden Dialkylether und Wasser zu bilden und

(2) das dabei entstehende Dialkyletherprodukt von Wasser und nicht umgesetztem Material fraktioniert,

c) das Dialkyletherprodukt an einem Punkt oberhalb der Destillationsreaktionszone aus dem Destillationssäulenreaktor abzieht und einen Teil davon als Rückfluß zum Destillationssäulenreaktor zurückführt,

d) das Wasser in einer Destillationszone unterhalb der Destillationsreaktionszone von den nicht umgesetzten Materialien trennt und

e) das Wasser als Bodenstrom aus dem Destillationssäulenreaktor abzieht.

**9.** Verfahren nach Anspruch 1 oder 2, bei dem der Alkohol n-Butanol und der Dialkylether Di-n-butylether ist und (1) der erste, den Di-n-butylether enthaltende Strom an einem Punkt unterhalb der Destillationsreaktionszone aus dem Destillationssäulenreaktor abgezogen wird und (2) nicht umgesetztes n-Butanol und Wasser in Schritt (d) als Produkt abdestilliert wird, das ein Azeotrop aus n-Butanol, Di-n-butylether und Wasser enthält.

**10.** Verfahren nach Anspruch 9, bei dem der Druck in der Säule im Bereich von 0 bis 689 kPa g (0 bis 100 psig) liegt.

**11.** Verfahren nach Anspruch 8 oder 9, bei dem die Temperatur im Bereich von 90 bis 150°C liegt.

**12.** Verfahren nach einem der Ansprüche 9 bis 11, bei dem der Druck in der Säule im Bereich von 0 bis 275,6 kPa g (0 bis 40 psig) und die Temperatur im Bereich von 90 bis 120°C liegt.

**13.** Verfahren nach einem der Ansprüche 1 bis 12, bei dem die Temperatur in der Destillationsreaktionszone der Siedepunkt des Reaktionsgemischs bei den dort herrschenden Druckbedingungen ist.

**14.** Verfahren zur Herstellung von Di-n-Butylether nach einem der Ansprüche 9 bis 13, bei dem man

a) einen n-Butanol enthaltenden Strom in einen Destillationssäulenreaktor in eine Beschickungszone einspeist,

b) gleichzeitig

(1) den das n-Butanol enthaltenden Strom mit einer festen, sauren, katalytischen Destillationsstruktur in Festbettform in einer Destillationsreaktionszone bei einem Druck zwischen 0 bis 689 kPa g (0 bis 100 psig) und einer Temperatur zwischen 90 und 150°C in Kontakt bringt und dadurch mindestens einen Teil des n-Butanols katalytisch zur Umsetzung bringt, um Di-n-butylether und Wasser zu bilden, und

(2) das dabei entstehende Di-n-butyletherprodukt von Wasser und nicht umgesetztem Material fraktioniert,

c) Di-n-butyletherprodukt an einem Punkt unterhalb der Destillationsreaktionszone aus dem Destillationssäulenreaktor abzieht,

d) einen ein Azeotrop aus n-Butanol, Di-n-butylether und Wasser enthaltenden Strom abdestilliert,

e) den abdestillierten Strom als Zweiphasengemisch, das aus einer oberen, hauptsächlich n-Butanol und Di-n-butylether enthaltenden Phase und einer unteren wasserhaltigen Phase besteht, kondensiert und akkumuliert,

f) die hauptsächlich Wasser enthaltende Bodenphase abzieht und etwa darin befindlichen Alkohol zurückgewinnt und

g) die obere, hauptsächlich n-Butanol und Di-n-butylether enthaltende Phase abzieht und einen Teil davon als Rückfluß zum Destillationssäulenreaktor zurückführt.

**Revendications**

1. Procédé pour la production d'éther dialkylique par déshydratation d'un alcool, comprenant les étapes de :

   (a) introduction d'un courant contenant un alcool alkylique dans une zone d'alimentation d'un réacteur à colonne de distillation,

   (b) concouramment :

   (1) mise en contact de ce courant avec une structure de distillation catalytique acide solide à lit fixe dans une zone de réaction avec distillation, de façon à faire réagir catalytiquement au moins une partie de cet alcool pour former de l'éther dialkylique correspondant et de l'eau, et

   (2) fractionnement de l'éther dialkylique produit résultant de l'eau et de la matière n'ayant pas réagi,

   (c) soutirage de l'éther dialkylique produit du réacteur à colonne de distillation en tant que premier courant contenant de l'éther dialkylique pratiquement pur,

   (d) soutirage de l'eau et de la matière n'ayant pas réagi de la colonne de distillation en tant que second courant, dans lequel l'un des ces premier et second courants est soutiré en tant que fraction de tête, et

   (e) recyclage d'une partie de ce premier courant, de ce second courant ou de ce premier courant et de ce second courant vers le réacteur à colonne de distillation.

2. Procédé suivant la revendication 1, dans lequel cet alcool constitue 40 à 100% du courant d'alimentation.

3. Procédé suivant les revendications 1 ou 2, dans lequel cet alcool est choisi dans le groupe consistant en méthanol, éthanol ou propanol, et (1) ce premier courant est soutiré de ce réacteur à colonne de distillation en un point situé au-dessus de cette zone de réaction avec distillation, (2) cette eau est séparée de cette matière n'ayant pas réagi dans une zone de distillation située en-dessous de cette zone de réaction avec distillation et soutirée en tant que fraction de queues.

4. Procédé suivant la revendication 3, dans lequel cette structure de distillation catalytique acide solide consiste en un tamis moléculaire acide.

5. Procédé suivant les revendications 3 ou 4, dans lequel la pression dans la colonne est comprise dans la gamme de 137,8 à 6890 kPa.G (20 à 1.000 psig).

6. Procédé suivant l'une quelconque des revendications 3 à 5, dans lequel la température est comprise dans la gamme de 70°C à 300°C.

7. Procédé suivant l'une quelconque des revendications 3 à 6, dans lequel la pression dans la colonne est comprise dans la gamme de 344,5 à 4134 kPa.G (50 à 600 psig) et la température est comprise dans la gamme de 90°C à 250°C.

8. Procédé pour la production d'éther diméthylique, diéthylique ou dipropylique suivant l'une quelconque des revendications 1 à 7, comprenant les étapes de :

   (a) introduction d'un courant contenant un alcool alkylique choisi dans le groupe consistant en méthanol, éthanol ou propanol, dans une zone d'alimentation d'un réacteur à colonne de distillation,

   (b) concouramment :

   (1) mise en contact de ce courant contenant cet alcool avec une structure de distillation catalytique acide solide à lit fixe dans une zone de réaction avec distillation, à une pression comprise dans la gamme de 137,8 à 6890 kPa.G (20 à 1.000 psig) et à une température comprise entre 70°C et 300°C, de façon à faire réagir catalytiquement au moins une partie de cet alcool pour former de l'éther dialkylique correspondant et de l'eau, et

   (2) fractionnement de l'éther dialkylique produit résultant de l'eau et de la matière n'ayant pas réagi,

   (c) soutirage de cet éther dialkylique produit de ce réacteur à colonne de distillation en un point situé au-dessus de cette zone de réaction avec distillation et renvoi d'une partie de celui-ci vers ce réacteur à colonne de distillation en tant que reflux,

(d) séparation de l'eau et ces matières n'ayant pas réagi dans une zone de distillation située en-dessous de cette zone de réaction avec distillation, et

(e) soutirage de cette eau de ce réacteur à colonne de distillation en tant que fraction de queues.

**9.** Procédé suivant les revendications 1 ou 2, dans lequel cet alcool est le n-butanol et cet éther dialkylique est l'éther di-n-butylique, et (1) ce premier courant contenant cet éther di-n-butylique est soutiré de ce réacteur à colonne de distillation en un point situé au-dessous de cette zone de réaction avec distillation, et (2) du n-butanol n'ayant pas réagi et de l'eau sont soutirés dans l'étape (d) en tant que fraction de tête comprenant un azéotrope de n-butanol, d'éther di-n-butylique et d'eau.

**10.** Procédé suivant la revendication 9, dans lequel la pression dans la colonne est comprise dans la gamme de 0 à 689 kPa.G (0 à 100 psig).

**11.** Procédé suivant les revendications 8 ou 9, dans lequel la température est comprise dans la gamme de 90°C à 150°C.

**12.** Procédé suivant l'une quelconque des revendications 9 à 11, dans lequel la pression dans la colonne est comprise dans la gamme de 0 à 275,6 kPa.G (0 à 40 psig) et la température est comprise dans la gamme de 90°C à 120°C.

**13.** Procédé suivant l'une quelconque des revendications 1 à 12, dans lequel la température dans cette zone de réaction avec distillation est le point d'ébullition du mélange réactionnel dans les conditions de pression qui y règnent.

**14.** Procédé pour la production d'éther di-n-butylique suivant l'une quelconque des revendications 9 à 13, comprenant les étapes de :

(a) introduction d'un courant contenant du n-butanol dans une zone d'alimentation d'un réacteur à colonne de distillation,

(b) concouramment :

(1) mise en contact de ce courant contenant ce n-butanol avec une structure de distillation catalytique acide solide à lit fixe dans une zone de réaction avec distillation, à une pression comprise dans la gamme de 0 à 689 kPa.G (0 à 100 psig) et à une température comprise entre 90°C et 150°C, de façon à faire réagir catalytiquement au moins une partie de ce n-butanol pour former de l'éther di-n-butylique et de l'eau, et

(2) fractionnement de l'éther di-n-butylique produit résultant de l'eau et de la matière n'ayant pas réagi,

(c) soutirage de l'éther di-n-butylique produit de ce réacteur à colonne de distillation en un point situé au-dessus de cette zone de réaction avec distillation,

(d) soutirage d'un courant de tête comprenant un azéotrope de n-butanol, d'éther di-n-butylique et d'eau:

(e) condensation et accumulation de ce courant de tête sous forme de mélange à deux phases ayant une phase supérieure consistant principalement en n-butanol et en éther di-n-butylique, et en une phase inférieure comprenant de l'eau,

(f) soutirage de cette phase inférieure comprenant principalement de l'eau et récupération de l'alcool éventuellement contenu dans celle-ci, et

(g) soutirage de cette phase supérieure comprenant principalement du n-butanol et de l'éther di-n-butylique et renvoi d'une partie de celle-ci vers ce réacteur à colonne de distillation en tant que reflux.

14

FIG. 1

BUTYL ETHER
N-BUTANOL
H₂O

CONDENSER    *121*

*111*    *112*

*122*

*113*    ACCUMULATOR    *114*

*119*    N-BUTANOL
BUTYL ETHER    *115*

N-BUTANOL    *120*

*110*

*118*

*123*

*116*    REBOILER

*117*

BUTYL ETHER

FIG. 2

16